# EUROPEAN PATENT SPECIFICATION

(11) **EP 3 376 332 B1**
(45) Date of publication and mention of the grant of the patent: **01.09.2021**
(21) Application number: 17861204.0
(22) Date of filing: 25.04.2017
(51) Int. Cl.: G05D 1/10, B64C 39/02

(54) **AUTOMATED AGRICULTURAL POLLINATION METHOD BASED ON MICRO FLYING ROBOT**
VERFAHREN ZUR AUTOMATISCHEN LANDWIRTSCHAFTLICHEN BESTÄUBUNG AUF DER BASIS EINES MIKROLUFTFAHRZEUGS
PROCÉDÉ DE POLLINISATION AGRICOLE AUTOMATISÉE BASÉ SUR UN MICROROBOT VOLANT

(30) Priority: 23.01.2017 CN 201710049073
(43) Date of publication of application: 19.09.2018
(73) Proprietor: Dongguan University of Technology, Dongguan, Guangdong 523808 (CN); Chen, Yi, Dongguan, Guangdong 523808 (CN); Li, Yun, Dongguan, Guangdong 523808 (CN); Yu, Hongnian, Dongguan, Guangdong 523808 (CN)
(72) Inventor: CHEN, Yi, Dongguan, Guangdong 523808 (CN); LI, Yun, Dongguan, Guangdong 523808 (CN); YU, Hongnian, Dongguan, Guangdong 523808 (CN)
(74) Representative: Canzler & Bergmeier Patentanwälte Partnerschaft mbB
(86) International application number: PCT/CN2017/081877
(87) International publication number: WO 2018/133240

(56) References cited:
- WO-A1-2016/196616
- CN-A- 105 993 393
- CN-U- 204 733 723
- JP-A- 2011 200 196
- KR-B1- 101 668 635
- KARTHIK DANTU ET AL: "A comparison of deterministic and stochastic approaches for allocating spatially dependent tasks in micro-aerial vehicle collectives", INTELLIGENT ROBOTS AND SYSTEMS (IROS), 2012 IEEE/RSJ INTERNATIONAL CONFERENCE ON, IEEE, 7 October 2012 (2012-10-07), pages 793-800, XP032288079, DOI: 10.1109/IROS.2012.6386233 ISBN: 978-1-4673-1737-5

## Description

### TECHNICAL FIELD

The present invention relates to a method of pollination for plants, and specifically relates to a method of automatic agricultural pollination based on a micro air vehicle (MAV).

### BACKGROUND

WO 2016/196616 discloses a method of pollinating a plant. According to this method, plant data regarding a plant having flowers are received by a processing circuit. Operation of a robotic device for selectively pollinating a portion of the plurality of flowers based on the plant data is controlled by the processing circuit. The robotic device includes sensors configured to acquire plant data, a pollination device configured to pollinate flowers of a plant, a collection device configured to collect pollen, and a pollination prevention device, which is configured to prevent a flower from being pollinated. It is a disadvantage that the plant data have to be acquired by the robotic device as communication and correct acquisition of data might be difficult within its working area.

"Pollination" is always strongly influencing a correlation both inside and outside an ecological system, a stability of the ecological system and a genetic diversity of phytocoenosium. Research shows that about one third of the food for human beings grows depending on pollination by animals and insects, among which honeybees are one of the main pollinators. In particular, a main reason, the global epidemic of Colony Collapse Disorder (CCD), has seriously influenced the food safety of human beings. The present invention proposes an automatic solution of agricultural pollination by an autonomous micro hummingbird air vehicle.

### SUMMARY OF THE INVENTION

An object of the present invention is to provide a method of automatic agricultural pollination based on a micro air vehicle with high efficiency and good effect, in order to solve the problems stated in the above-described background.

In order to achieve the above-described object, the present invention provides a technical solution below:
A method of automatic agricultural pollination based on a micro air vehicle, has detailed steps as follows:
   (1) in a current pollination working area j, a central control system CCS assigning a work task to a micro air vehicle pollinator µMAVi;
   (2) capturing a data of flower via a camera and a data acquisition device;
   (3) processing an original data collected from a specific data source, and then screening, filtering and preprocessing the original data;
   (4) according to a data flow, performing an operation of recognizing the flower;
   (5) assessing whether a designated flower is successfully recognized or not;
   (6) the micro air vehicle pollinator µMAVi pollinating the designated flower;
   (7) assessing a pollination result and an effectiveness of the micro air vehicle pollinator, named µMAVi, determining whether to end a work of the current working area j and to enter into the next working area j+1 or not; and
   (8) if the assessment being negative, then performing the pollination again; and if the assessment being positive, then entering into the next working area j+1, and repeating steps 1 to 7.

A sphere working range for the air vehicle is formed with a point O as the center of the camera and an effective working distance of the camera as a radius.

The work task in the step (1) comprises pollinating flowers of a specific type of grain crop, a type of the flowers and a number of the flowers.

The data of flower in the step (2) is a photo of the flower.

Comparing with the prior art, the present invention has beneficial effects: The present invention is more efficient in an automatic process, reducing an operating cost at the same time, being advantageous for propelling agricultural intellectualization, and the present invention can provide a higher efficiency and a better pollination quality and improve an agricultural productivity.

### BRIEF DESCRIPTION OF THE DRAWINGS

- **FIG. 1**: is a schematic diagram of a method process of the present invention.
- **FIG. 2**: is a schematic diagram of a working principle of the present invention.

### DETAILED DESCRIPTION OF THE PREFERRED EMBODIMENT

The technical solution of the present application is described in detail below by combining with specific implementation.

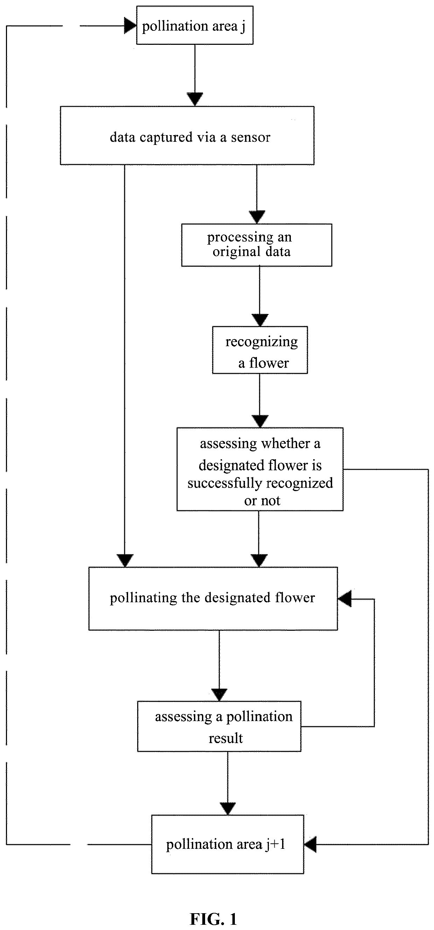

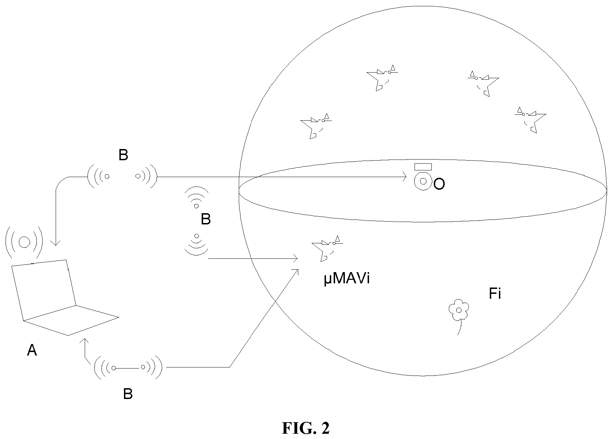

Referring to FIG. 1 and FIG. 2, a method of automatic agricultural pollination based on a micro air vehicle, has detailed steps as follows:
(1) in a current pollination working area j, a work task is assigned by a central control system (CCS) to a micro air vehicle pollinator µMAVi; and the work task comprises pollinating flowers of a specific type of crop, a type of the flowers and a number of the flowers;
(2) a data of flower is captured via a camera and a data acquisition device; and the data of flower is a photo of the flower;
(3) an original data collected from a specific data source is processed, and then the original data was screened, filtered and preprocessed;
(4) according to a data flow, an operation of recognizing the flower is performed;
(5) whether a designated flower is successfully recognized or not is assessed;
(6) the designated flower is pollinated by the micro air vehicle pollinator µMAVi;
(7) a pollination result and an effectiveness of the micro air vehicle pollinator µMAVi are assessed, whether to end a work of the current working area j and to enter into the next working area j+1 or not is determined; and
(8) if the assessment is negative, then the pollination will be performed again; and if the assessment is positive, then the micro air vehicle pollinator µMAVi will enter into the next working area j+1, and steps 1 to 7 will be repeated.

In application scenarios that require a large-scale pollination activity, such as a production base of grain crop, a botanical garden and an orchard, or a production base of commercial seeds and the like, the method of automatic agricultural pollination based on the micro air vehicle of the present invention provides a "substantial" food supply method.

A sphere working range is formed with a point O as the center of the camera and an effective working distance of the camera as a radius, µMAVi is a micro air vehicle system and Fi is the flower of the crop.

The invention is more efficient in an automatic process, and reduces an operating cost at the same time, no matter the number of workers increases or decreases. Then agriculture workers, no matter being substituted by other workers or robots, can freely focus on work on which they can exert greater influence and can execute a higher-value work. Besides, requirements for the micro air vehicle for autonomous agricultural pollination create extra work, for example maintenance of the micro air vehicle system for autonomous agricultural pollination. Additionally, an agriculture system will more rely on the micro air vehicle for autonomous agricultural pollination, which is advantageous for propelling agricultural intellectualization.

From an economic point of view, the micro air vehicle for autonomous agricultural pollination provides a higher efficiency and a better pollination quality, and improves an agricultural productivity at the same time. The micro air vehicle system for autonomous agricultural pollination will directly influence the grain production, in order to improve a farming efficiency in the future. For example, in 2009, CCD had influenced 24% of grain crop based on a total yield of crop of UK; and in 2013, CCD had influenced products with value of 4 billion dollars in United States. Products of the micro air vehicle for autonomous agricultural pollination will accelerate a development of other industrial products, such as an advanced motor and MEMS system, and will enrich manufacturers or suppliers of advanced materials and industrial engineering.

In order to adjust to the requirements for efficient agricultural automatization in the future, the micro air vehicle for autonomous agricultural pollination, as a substitute for natural pollinators, such as honeybee, insect and the like, can act as an artificial pollinator in an industry of intelligent agriculture, reducing a problem that an ecological correlation, protection and stability of an ecological system, a heritable variation of phytocoenosium of crop, and a diversity, a specialization and an evolution of flowers are seriously influenced due to a decrease in a population quantity of natural pollination medium. The present invention is more efficient in the automatic process, reducing the operating cost at the same time, being advantageous for propelling agricultural intellectualization, and the present invention can provide a higher efficiency and a better pollination quality and improve the agricultural productivity.

The preferred implementation of the present application is described in detail above, but the present application isn't limited by the above-described implementation. Within the scope of knowledge owned by a person skilled in the art, a plurality of variations can also be made.

## Claims

1. A method of automatic agricultural pollination based on an air vehicle, the method having detailed steps as follows:
(1) in a current pollination working area (j), a central control system (CCS) assigning a work task to an air vehicle pollinator (µMAVi);
(2) capturing a data of flower (Fi) via a camera and a data acquisition device and the data of flower (Fi) is a photo of a flower (Fi);
(3) processing an original data collected from a specific data source, and then screening, filtering and preprocessing the original data;
(4) according to a data flow, performing an operation of recognizing the flower (Fi);
(5) assessing whether a designated flower (Fi) is successfully recognized or not;
(6) the air vehicle pollinator (µMAVi) pollinating (6b) the designated flower (Fi);
(7a) assessing a pollination result and an effectiveness of the air vehicle pollinator (µMAVi);
(7b) determining whether to end a work of the current working area (j) and to enter into the next working area (j+1) or not; and
(8a) if the assessment being negative, then performing the pollination again,
(8b) if the assessment being positive, then entering into the next working area (j+1), and repeating steps 1 to 7b and
**characterized in that**
- a sphere working range for the air vehicle is formed with a point (O) as the center of the camera and an effective working distance of the camera as a radius;
- the air vehicle is an autonomous micro hummingbird air vehicle and
- the work task in the step (1) comprises pollinating flowers (Fi) of a specific type of grain crop, a type of the flowers (Fi) of the grain crop and a number of the flowers (Fi) of the grain crop.

## Patentansprüche

1. Verfahren der automatischen landwirtschaftlichen Bestäubung, basierend auf einem Luftfahrzeug, wobei das Verfahren die folgenden detaillierten Schritte aufweist:
(1) Zuweisen in einem aktuellen Bestäubungsarbeitsbereich (j) durch ein zentrales Steuerungssystem (CCS) einer Arbeitsaufgabe einem Luftfahrzeug-Bestäuber (µMAVi);
(2) Erfassen von Blütendaten (Fi) über eine Kamera und eine Datenerfassungsvorrichtung, wobei die Blütendaten (Fi) ein Foto der Blüte (Fi) sind;
(3) Verarbeiten von Originaldaten von einer bestimmten Datenquelle und dann Sieben, Filtern und Vorverarbeiten der Originaldaten;
(4) gemäß einem Datenfluss Durchführen eines Vorgangs zum Erkennen der Blüte (Fi);
(5) Bewerten, ob eine bezeichnete Blüte (Fi) erfolgreich erkannt wurde oder nicht;
(6) Bestäuben (6b) der bezeichneten Blüte (Fi) durch den Luftfahrzeug-Bestäuber (µMAVi);
(7a) Bewerten eines Bestäubungsergebnisses und einer Effektivität des Luftfahrzeug-Bestäubers (µMAVi);
(7b) Bestimmen, ob eine Arbeit an dem aktuellen Arbeitsbereich (j) zu beenden ist, und in den nächsten Arbeitsbereich (j+1) einzutreten ist oder nicht; und
(8a) wenn die Bewertung negativ ist, dann erneutes Durchführen der Bestäubung,
(8b) wenn die Bewertung positiv ist, dann Eintreten in den nächsten Arbeitsbereich (j+1) und Wiederholen der Schritte 1 bis 7b, und
**dadurch gekennzeichnet, dass**
- ein Kugelarbeitsbereich für das Luftfahrzeug ausgebildet ist mit einem Punkt (O) als die Mitte der Kamera und einem effektiven Arbeitsabstand der Kamera als Radius;
- das Luftfahrzeug ein autonomes Mikro-Kolibri-Luftfahrzeug ist und
- die Arbeitsaufgabe im Schritt (1) das Bestäuben von Blüten (Fi) einer bestimmten Art von Getreidepflanzen, einer Art der Blüten (Fi) der Getreidepflanzen und einer Anzahl der Blüten (Fi) der Getreidepflanzen umfasst.

## Revendications

1. Procédé de pollinisation agricole automatique basée sur un véhicule aérien, le procédé comprenant des étapes détaillées comme suit :
(1) dans une zone de travail de pollinisation actuelle (j), un système de commande central (CCS) attribue une tâche de travail à un pollinisateur sur véhicule aérien (µMAVi) ;
(2) capturer des données de fleur (Fi) via une caméra et un dispositif d'acquisition de données et les données de fleur (Fi) sont une photo d'une fleur (Fi) ;
(3) traiter des données originales collectées à partir d'une source de données spécifique, puis cribler, filtrer et prétraiter les données originales ;
(4) selon un flux de données, effectuer une opération d'identification de la fleur (Fi) ;
(5) évaluer si une fleur (Fi) désignée est identifiée avec succès ou non ;
(6) le pollinisateur sur véhicule aérien (µMAVi) pollinise (6b) la fleur (Fi) désignée ;
(7a) évaluer un résultat de pollinisation et une efficacité du pollinisateur sur véhicule aérien (µMAVi) ;
(7b) déterminer s'il faut mettre fin à un travail dans la zone de travail actuelle (j) et entrer dans la zone de travail (j+1) suivante ou non ; et
(8a) si l'évaluation est négative, alors effectuer à nouveau la pollinisation,
(8b) si l'évaluation est positive, alors entrer dans la zone de travail (j+1) suivante et répéter les étapes 1 à 7b, et
**caractérisé en ce que**
- une zone de travail sphérique pour le véhicule aérien est formée avec un point (O) comme le centre de la caméra et une distance de travail effective de la caméra comme rayon ;
- le véhicule aérien est un véhicule aérien autonome à micro-colibri et
- la tâche de travail dans l'étape (1) comprend la pollinisation de fleurs (Fi) d'un type spécifique de culture céréalière, un type de fleurs (Fi) de la culture céréalière et un nombre de fleurs (Fi) de la culture céréalière.
